# EUROPEAN PATENT SPECIFICATION

(11) **EP 4 507 591 B1**
(45) Date of publication and mention of the grant of the patent: **08.07.2026**
(21) Application number: 23718064.1
(22) Date of filing: 14.04.2023
(51) Int. Cl.: A61B 17/15, A61B 17/80, A61B 17/88

(54) **PATIENT-SPECIFIC IMPLANT FOR A REPOSITION OSTEOTOMY**
PATIENTENSPEZIFISCHES IMPLANTAT FÜR EINE REPOSITIONSOSTEOTOMIE
IMPLANT SPÉCIFIQUE À UN PATIENT POUR UNE OSTÉOTOMIE DE REPOSITIONNEMENT

(30) Priority: 14.04.2022 NL 2031583
(43) Date of publication of application: 19.02.2025
(73) Proprietor: Cadskills BV, 9051 Gent (BE)
(72) Inventor: MOMMAERTS, Maurice Yves, 9830 Sint-Martens-Latem (BE); VAN DEN BOGAERT, Gert, 2200 Noorderwijk (BE); WINNOCK DE GRAVE, Philip, 9831 Deurle (BE); HUYS, Stijn, 2830 Willebroek (BE); LIPPENS, Alexander, 9270 Laarne (BE)
(74) Representative: De Clercq & Partners
(86) International application number: PCT/IB2023/053825
(87) International publication number: WO 2023/199276

(56) References cited:
- EP-A1- 3 845 153
- WO-A2-02/071924
- DE-U1- 202011 050 305

## Description

### BACKGROUND OF THE INVENTION

The invention relates to an implant, made-to-measure for a patient, which can be placed in an osteotomy (bone incision) in a long bone in order to be able to modify the mutual orientation of the bone portions located on either side of the bone incision. Here, it relates to a long bone in a supporting limb of a mammal, in particular a human being. The two bone portions can then be tilted to a desired mutual orientation by tilting at the location of a hinge created by the bone incision (realignment) and then can be secured in this state by means of the implant. The invention further relates to an assembly of an implant of this kind and a guide for a tool for making the incision. The invention further relates to an assembly of an implant of this kind and a spreader for opening up the incision. The invention further relates to a process for the placement of an implant of this kind.

In the case of bowleg or knock-knee, a reposition osteotomy (saw incision with alteration of the position of the resultant bone segments) is often used around the knee (femur, tibia) or near the ankle or the hip with the aim of altering and improving the axis of the leg and thereby the state (alignment) of the leg in order to prevent or combat premature cartilage wear or degenerative arthrosis. As a result of arthrosis, loading-related pain in young patients will form the commonest complaint that results in the need for said realignment/reposition osteotomy.

There are two kinds of reposition osteotomy, namely one with a closed wedge and one with an open wedge. In the first case two incisions are made in the bone; these incisions are at an acute angle to each other and meet each other in an end strip where the bone can form a hinge. The wedge-shaped bone material between the two bone incisions is removed, after which the bone portions above and below the incisions are turned toward each other by tilting about the hinge and are fixed in that state. In the second case one cut is made, up to an end strip where the bone can form a hinge. Then the bone portions above and below the incision are turned away from each other to form a wedge-shaped open space. In that case too, the desired state is fixed, for which an implant can be used.

The invention relates to reposition osteotomy in a long bone, in particular a body-supporting bone, of the kind with an open wedge. This may for example be surgery in the femur or surgery in the tibia. It may be surgery in the vicinity of a joint, such as a knee joint. In particular, the invention provides an implant for fixation of the desired open state, as well as a method for making the bone incision and for placement of the implant.

EP3845153A1 relates to a surgical guide for osteotomy operations, in particular for the implantation of osteosynthesis plates.

### SUMMARY OF THE INVENTION

One aim of the invention is to provide an implant and/or assembly of the kind mentioned in the introduction with which the accuracy of a reposition osteotomy can be promoted.

One aim of the invention is to provide an implant and/or assembly of the kind mentioned in the introduction, which can be used conveniently and/or easily.

From one aspect, the invention provides an implant for placement in a wedge-shaped space in a bone obtained by osteotomy, wherein the implant is made as a single whole and comprises a middle portion and two arms connected thereto, extending from opposite sides of the middle portion, wherein the middle portion and the two arms define a U shape,
wherein the arms each have an upper edge and a lower edge for engaging with the cut surfaces of the bone that define the wedge-shaped space, and that converge in a direction away from the middle portion,
wherein the middle portion has an upper edge that is located lower than the upper edge of the adjacent portions of the arms and has a lower edge that is located higher than the lower edge of the adjacent portions of the arms for forming a recess above the middle portion and a recess below the middle portion.

The implant is provided thereby, at the location of the middle portion, with a portion or bridge between the arms, with narrowing in the vertical direction relative to the connecting arms, which offers advantages in interaction with a so-called retractor or bone spreader with spreading arms with which the incision that has been made in the bone is opened up to produce a wedge-shaped space tailored to the implant that is to be placed. The implant can then be placed while the bone spreader is still in place and in operation without the action thereof being adversely affected. The spreading arms then reach through the recesses above and below the middle portion. The retractor can be removed after placement of the implant, by withdrawing the spreading arms through the recesses.

The implant is preferably made of a biocompatible material (such as titanium, tantalum, Vitallium, PEEK) or a bone substitute such as a 3D-printed calcium phosphate derivative, bioprinted bone or bioprinted cartilage.

The middle portion and the directly adjacent portions of the arms may define an H shape, wherein the middle portion forms the bridge of the H.

In one embodiment, the distance from the upper edge of the middle portion to the upper edge of the adjacent portions of the arms and the distance from the lower edge of the middle portion to the lower edge of the adjacent portions of the arms are approximately equal.

For securing the implant on the bone the arms may be provided, in the portions adjacent to the middle portion, with pairs of fastening lips, formed as one whole therewith, extending transversely from the arms, respectively upwards and downwards, which are provided with holes for fasteners for securing the implant on the cortex of the bone. The arms of the implant will take the axial load and thus prevent closing up of the osteotomy, while the fastening lips will take the lateral forces and thus prevent the implant coming out of the osteotomy.

The absorption of force can be promoted if at least one of the arms is provided with a second pair of fastening lips positioned closer to the end of the respective arm.

If the fastening lips are displaced relative to the respective arm, in a direction turned away from the space enclosed by the arms, the middle portion and the two arms may remain within the contour of the bone, whereas the lips abut against the external surface of the bone. As a result, less foreign material is present on the outside of the piece of bone and there is less likelihood of subcutaneous irritation for the patient.

The arms have an internal surface and an external surface, wherein the external surface of the arms is preferably convex, considered in the direction of extension of the arms. The implant then follows the arched shape of the osteotomy platform. The internal surface of the arms is preferably concave, considered in the direction of extension of the arms. Over their length, the arms may have an at least approximately constant thickness, tailored to the thickness of the cortex of the bone at the location of the bone incision.

In one embodiment, at least one arm is provided, on the internal surface facing the other arm, with a porous structure with interconnecting pores, which may come into contact with the cortex and marrow, and allows ingrowth of bone tissue. The outer side of the arms forms a massive layer, which is formed as one whole with the porous layer. The porous structure may extend into at least one of the upper edge and lower edge of the respective arm, preferably into both edges. Through the ingrowth of bone tissue in the porous structure from the cut surface of the bone at the location of the cortex, connection of the implant with the bone will be promoted (osteogenesis/bone healing). Moreover, owing to bone ingrowth and the resultant improvement of the implant-bone connection, the loading on the bone screws of the implant may be reduced. This might counteract the development of an abnormal state in the bone. The porous layer on the inside of the arms may extend up to the distance of the ends of the arms, where the massive portion of the arms, which is located on the outer side of the porous portion, surrounds the porous portion in the distal direction, as a kind of nose.

The middle portion may also be provided, on the internal surface facing the space delimited by the arms, with a porous structure that allows ingrowth of bone tissue, when the bone tissue has been able to develop that far.

At least one of the arms may be provided with a passage to the space delimited by the arms that is located between the upper edge and lower edge going through there. Through this passage, minerals, growth factors and/or stem cells may be introduced into said space. This may optionally also take place through the aforementioned recesses in the middle portion.

From a further aspect, the invention provides an assembly of an implant according to the invention and a bone spreader, wherein the bone spreader has two spreading arms and spreading jaws located on the end thereof for forcing the cut surfaces of the bone apart, wherein the recesses above and below the middle portion of the implant are dimensioned to provide space for the respective spreading arms. The recesses are preferably dimensioned above and below the middle portion for the spreading jaws to pass through them. The width of the recesses is preferably greater than the width of the spreading arms and/or the width of the spreading jaws. The result is that with the bone spreader placed in the spread state, wherein a wedge-shaped space is created in the bone that is tailored to the implant, placement of the implant in the desired position in/on the bone is facilitated. The placed implant will also make withdrawal of the spreading jaws from the wedge-shaped space possible.

In one embodiment, in which the spreading arms are joined together by a hinge but do not cross each other such as is the case with shears, the implant has, in at least one of the arms, a largest height (in particular the distance between the ends of two lips located opposite each other) that is less than the distance between the hinge and the spreading jaws. As a result the implant may, in a horizontal position of one arm, be fed with that arm between the spreading arms until the middle portion is located between the spreading arms. Then the implant can be tilted toward the bone to a state coinciding with the working state and can be introduced into the wedge-shaped space in the bone, while the bone spreader continues to function. After the implant has been fastened to the bone, the bone spreader can be removed, wherein the spreading jaws are pulled back through the recesses of the middle portion.

In another embodiment, the bone spreader has two parallel spreading arms with spreading jaws, and the distance between the spreading arms is adjustable, for example by means of a rack mechanism. In one embodiment, the spreading jaws are transverse to the spreading arms, so that after placement on the bone, the spreading arms are directed tangentially relative to the bone surface. In another embodiment, the spreading jaws lie in line with or parallel to the spreading arms, so that after placement on the bone, the spreading arms are directed radially.

Also described, but not claimed, is a guide for a saw for osteotomy, comprising a frame with an internal surface for engaging with a bone surface and with a number of fastening holes for fastening the guide on a bone, wherein the frame has two guide parts, which between them define a slot-shaped guide space for the saw, wherein the guide parts have an external surface turned away from the internal surface, which can serve as a stop surface for a saw, and has a concave shape. Owing to the concave shape, the saw will be able to come closer to the opposite, closed region of the cortex (residual lateral bone wall). This would facilitate opening up of the bone incision.

Also described, but not claimed, is a guide for a saw for osteotomy, comprising a frame with an internal surface for engaging with a bone surface and with a number of fastening holes for fastening the guide on a bone, wherein the frame has two guide parts, which between them define a slot-shaped guide space for the saw, wherein the slot-shaped guide space, at least partly and at least on one side, is open sideways and forms a side-slot. As a result, a large degree of freedom in saw movements is offered, promoting controllability of the osteotomy. This is particularly the case if there are these side-slots on both sides. The cortex can then be reached easily with the saw blade to the left and right. Moreover, it is possible that one or each side-slot opens freely in the inside of the frame.

In a further example, the frame is provided, directly above or directly below the guide parts, with a handle for positioning the guide on the bone.

Also described, but not claimed, is a method for carrying out an operation for correction of the state of a center line of a long bone of a leg supporting a body of a mammal, wherein:
- a saw guide is fastened on the long bone,
- with a saw guided by the guide, an incision is made in the bone at a place, direction and depth predetermined for the respective surgery,
- with a bone spreader, the bone portions above and below the incision are forced apart to form a wedge-shaped space of a size predetermined for the respective surgery, wherein the bone spreader has two spreading arms that can be forced apart,
- the bone spreader is secured in this state,
- an implant according to the invention is held ready,
- wherein the distance between the spreading arms where they enter the wedge-shaped space is greater than the height of the middle portion of the implant between the recesses,
- with a rotating motion in a plane that is transverse to the spreader arms, the implant is introduced with one arm between the spreading arms of the secured bone spreader, until the middle portion is located between the two spreading arms,
- the implant is tilted about a transverse axis on the plane in which the two spreading arms lie, in order to bring the arms of the implant to the level of the opening of the wedge-shaped space,
- the implant is pushed into the wedge-shaped space,
- the implant is fastened to the bone,
- the bone spreader is removed, wherein the spreading jaws are withdrawn through the recesses in the middle portion of the implant.

In one example, the distance between the spreading arms is greater than the largest dimension of the cross section of the middle portion of the implant between the recesses. The largest dimension may be the diagonal of a rectangular section.

In one example, the surgery is carried out in a proximal portion of the tibia of a supporting leg of a person, wherein the implant is introduced between the two spreading arms with the opening of the U directed downward.

In one example, the surgery is carried out in a distal portion of the femur of a supporting leg of a person, wherein the implant is introduced between the two spreading arms with the opening of the U directed upward.

### SUMMARY OF THE INVENTION

The aspects and features described in this description and the claims of the application and/or shown in the drawings of this application may where possible also be applied separately from one another. These separate aspects may form the subject matter of divisional patent applications directed at these. This applies in particular to the features and aspects that are described per se in the subclaims.

### BRIEF DESCRIPTION OF THE DRAWINGS

The invention will be explained on the basis of an exemplary embodiment presented in the appended drawings. In these:
Figs. 1A-C show a number of views of an exemplary embodiment of an implant according to the invention;
Figs. 2A-D show a number of views of an exemplary osteotomy guide as disclosed herein;
Fig. 3A shows an illustration of a first example of a bone spreader for use in a method as disclosed herein;
Figs. 3B and 3C show illustrations of two other embodiments of a bone spreader for use in a method as disclosed herein;
Figs. 4A and 4B show two views of the osteotomy guide in Figs. 2A-D fastened to a tibia for making a bone incision;
Figs. 5A-D show successive stages in the carrying out of a part of a method as disclosed herein, wherein use is made of a bone spreader according to Fig. 3B and the implant in Figs. 1A-C;
Figs. 6A-D show successive stages in the carrying out of a part of a method as disclosed herein, wherein use is made of a bone spreader according to Fig. 3C and the implant in Figs. 1A-C; and
Figs. 7A-C show a number of views of the implant in Figs. 1A-C after removal of the bone spreader.

### DETAILED DESCRIPTION OF THE DRAWINGS

For a description of the components that are discussed hereunder, for better understanding use is made of a reference above/below. This is indicated with V1 above and V2 below.

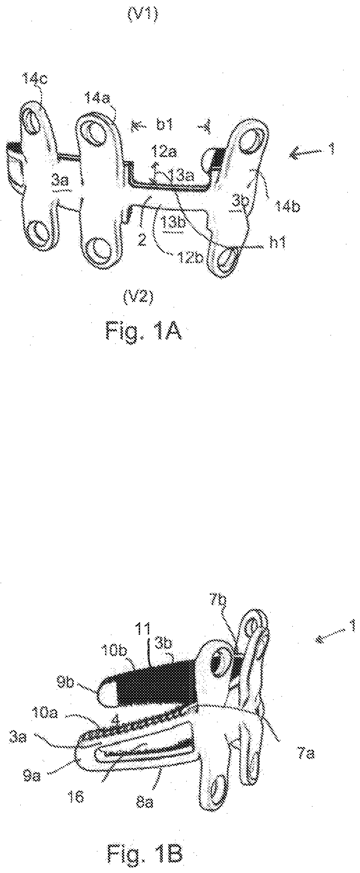

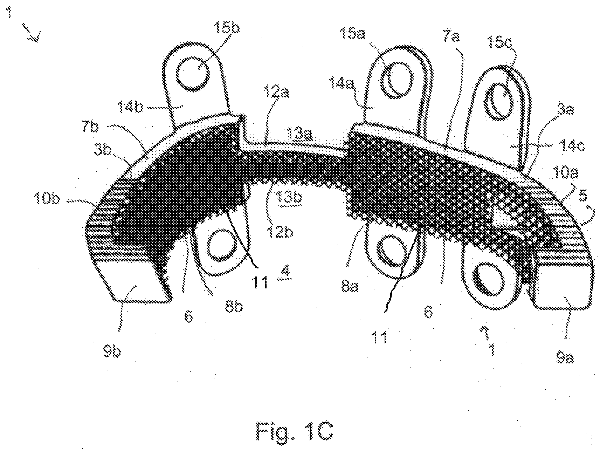

The wedge-shaped implant 1 in Figs. 1A-C is made of biocompatible material, such as titanium, tantalum, Vitallium, PEEK, or bone substitute. The implant 1 is formed as a single whole, by a 3D printing technique, to measure according to the unique anatomy of the portion of bone to be corrected and consequently is completely adapted to the pieces of bone that surround the bone incision and also ensures the exact opening of the previously calculated wedge.

The implant 1 comprises a middle portion 2 and two arms 3a and 3b extending therefrom, which define with the middle portion a U-shaped body with internal space 4, with a convex external surface 5 and a concave internal surface 6. The arms 3a and 3b are curved and strip-shaped and have respective upper edges 7a,7b and lower edges 8a,8b, which run toward ends 9a,9b so that implant 1 has a wedge shape in side view (Fig. 1B).

The upper edges 7a,7b and the lower edges 8a,8b are provided with asymmetric teeth 10a,10b, which extend as far as the ends 9a,b.

The middle portion 2 or bridge has an upper edge 12a and a lower edge 12b. These edges lie vertically backward relative to the upper and lower edges of the arms 3a,3b, so that recesses 13a,13b are formed, which have a height h1 and a width b1.

In the region of abutment of the arms 3a,3b on the middle region 2, the arms 3a,3b are formed with lips 14a,14b, which extend via a kink outside of the external surface 5, upward and downward. The lips are provided with holes 15a, 15b for fasteners, such as bone screws.

In this example the arm 3a is provided with an extra pair of lips 14c with fastening holes 15c, at a place between the lips 14a,b and the end of the arm 9a.

The front arm 3b is in this case somewhat shorter than the arm 3a to avoid conflict with the tuberositas tibiae, the insertion site of the patellar tendon, in the case of valgizing osteotomy. Most forces will come down on the longer arm, such as when walking.

On the inside of the implant 1, the arms 3a,b and the middle portion 2 are provided with a continuous porous layer 11, formed as one whole therewith, which defines almost the entire internal surface 6. The openings of the porous layer 11 are such as to allow bone growth through them. The porous layer 11 reaches into the upper edges 7a,b and into the lower edges 8a,b. The porous layer 11 reaches to the ends 9a,b, which are of massive material and close off/screen off the porous layer 11 distally and thus protect against damage during placement of the implant.

In this example, arm 3a is provided with a passage 16 going through the arm.

The implant 1 is made as a unique item, based on the desired dimensions and shape for the patient being treated, in particular the arm lengths and the wedge angle, as well as the locations of the fastening lips. Furthermore, the thickness of the arms is tailored to the thickness of the cortex of the bone, so that the porous layer makes functional contact with the cortex for the process of bone ingrowth. The cortex may in this case be a few mm thick, for example 3 mm. The thickness of the arms 3a,b, with massive portion and porous portion, may also be 3 mm. The thickness may also be greater, to allow the porous portion to be in contact with the marrow as well, so that it is easier for stem cells to enter the pores.

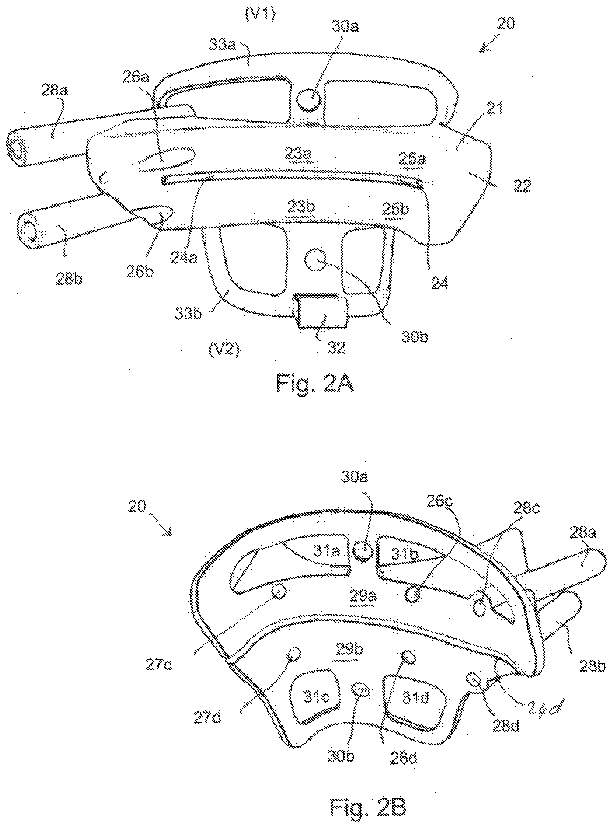

The osteotomy guide 20 in Figs. 2A-D is made of a material suitable for the function, for example plastic or metal, which does not have to be biocompatible. The guide 20 consists of a body 21 formed as a single whole. At the front, the body 21 comprises a flat block-like portion 22, defining an upper guide 23a and a lower guide 23b, which leave a saw slot 24, with access 24a, free between them.

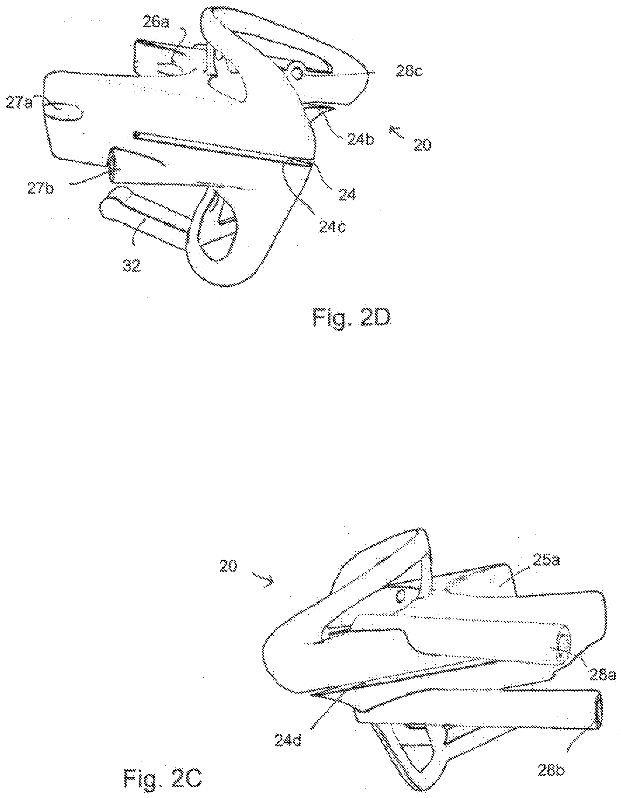

The front surfaces 25a,b of the upper guide 23a and lower guide 23b are concave. This shape is determined depending on which way the osteotomy is to extend in the bone. The front surfaces 25a,b form a stop for a projection on a saw to be used for the osteotomy, and determine the shape and position of the end of the incision thereby. As can be seen in Figs. 2C and 2D, for a large part the saw slot 24 is open sideways, at the location of 24c,d, into the rear end or inside end. The side-slots 24c,d do not reach the front surface 25.

The body 21 is formed above and below the portion 22 with portions 33a,b provided with recesses 31a-d. The recesses 31a-d limit the amount of material required and promote visual monitoring during use.

Between the recesses 31a and 31b there is a fastening hole 30a and between the recesses 31c and 31d there is a fastening hole 30b, provided for fastening on the bone.

At the front, the body is further provided with a handle 32 with which the guide 20 can be held in a controllable manner initially against the bone to be treated, until the guide 20 is fastened on the bone.

The body 21 is formed on the front side and/or sides with drilled passages, namely drilled passages 27a,b on one side and drilled passages 26a,b and 28a,b on the other side. These passages are provided so that when the guide is placed on the bone to be treated, holes are made at the precise places in the bone, in which after placement of the implant 1, the bone screws 17 that are to be fitted through the holes 15a,b,c in the lips 14a,b,c of the implant 1 can be applied, in the correct direction.

On the rear shown in Fig. 2B, the body 21 is divided by the outlet 24b of the slot 24. It can be seen that the drilled passages 26a,b come out in holes 26c,d, the drilled passages 27a,b in holes 27c,d and the drilled passages 28a,b in holes 28c,d.

The guide 20 is made as a unique item, tailored to the desired implant 1, based on the desired dimensions and shape for the patient being treated, particularly the shape of the back surface, the front surface and the location and direction of the drilled passages. The back surface is tailored to the bone to be treated so that it fits accurately on the bone surface.

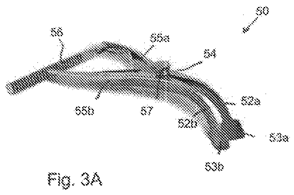

The bone spreader or retractor 50 in Fig. 3A comprises two arms 51a,b, which are hinged together at the location of 54 but do not cross each other, so that a movement of the proximal ends toward each other leads to a parting of the distal ends. On one side of the hinge 54, arm parts 52a,b extend in the distal direction to spreading jaws 53a,b, which are provided with stops 51a,b. On the opposite side of the hinge, arm parts 55a,b extend in the proximal direction to an adjusting strip 56. This adjusting strip 56 is provided with a size indication that is coordinated with the distance between the spreading jaws 53a,b. The distance that the arm parts 52a,d occupy at the location of the spreading jaws 53a,b can be secured with knob 57.

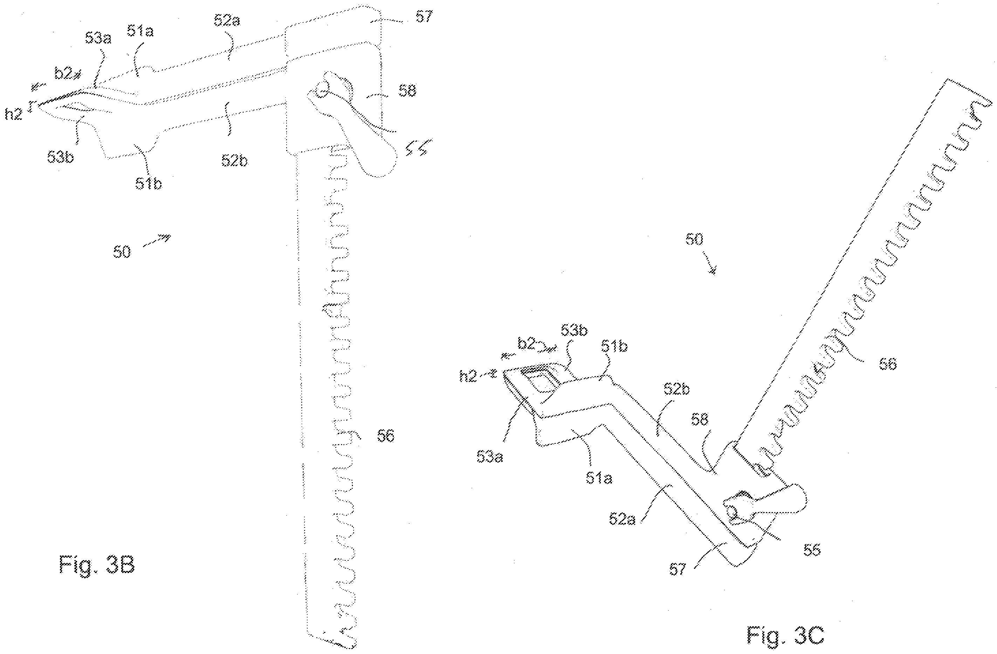

Fig. 3B shows another embodiment of a bone spreader 50, wherein the spreading arms 52a,b are parallel. The spreading arm 52a is fastened via block 57 on the upper end of a rack 56. The spreading arm 52b is applied conductively via block 58 on the rack 56. Within block 58 there is a gear wheel for movement of block 57 and therefore movement of the spreading arm 52b relative to spreading arm 52a. For this purpose, a crank mechanism 55 is provided, with which the position of the block 58 along the rack 56 can also be locked. The spreading jaws have a width b2 and a height h1, which are less than the width b1 and the height h1 of the recesses 13a,b.

The bone spreader 50 in Fig. 3C differs from that in Fig. 3B in that the spreading jaws 53a,b are not transverse to the rest of the spreading arms but extend into the extension thereof, displaced sideways but parallel thereto.

An example of an operation with placement of the implant described above in the proximal portion K of a tibia T will be described below.

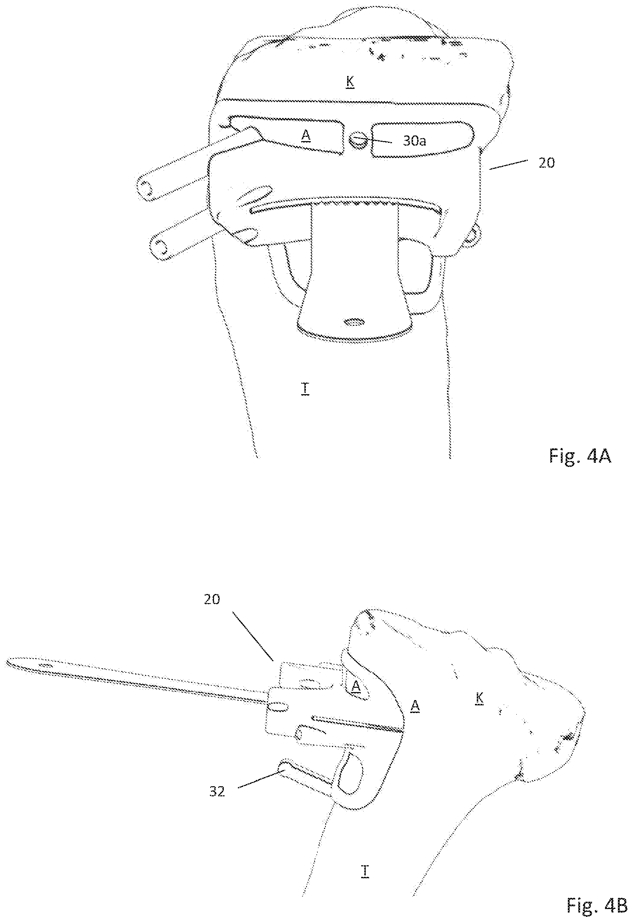

After the correction of the state of the bone that is necessary has been determined specifically for the patient and the required implant 1 and guide 20 have been designed and made, a start can be made with the placement and the fastening of the guide 20, see Figs. 4A and 4B.

The handle 32 is useful for this. Fastening screws are introduced via holes 30a,b to secure the guide 20 on the bone, against the bone surface A.

With a drill, holes are predrilled via the passages 26a,b, 27a,b and 28a,b, which are used later for fastening the implant 1.

With a bone saw provided with a stop, which is introduced into the slot 24, a limited incision is made in the bone, through the cortex and through the spongy region, to the desired, predetermined depth and over a predetermined portion of the cross section of the bone. Once the incision S is completed, the guide can be removed.

With an auxiliary tool, not shown, the bone incision is forced open, wherein the two bone portions delimiting the bone incision then hinge more or less about the still intact portion of the bone. The auxiliary tool may for example be a massive metal wedge, which is introduced with impact force. This as it were prepares the bone incision for a more accurate process of opening up the bone incision for the implant. A bone spreader shown in Figs. 3A-C can be used for this process.

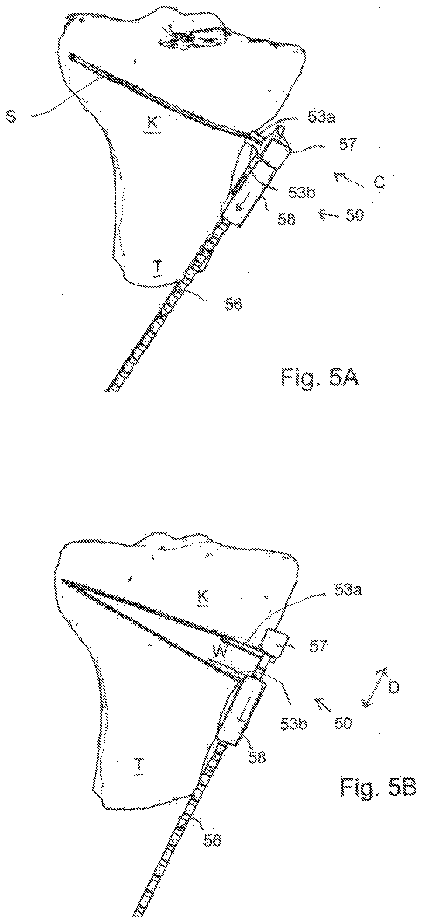

In Figs. 5A-F, the bone spreader 50 in Fig. 3B is then used. The bone spreader 50 is pressed with the spreading jaws 53a,b into the slot S, in direction C, until the stops 51a,b come up against bone surface A of head K. The spreading arms 52a,b are in this case directed tangentially relative to the bone.

Next, tool 55 (omitted here) is used for moving the blocks 57 and 58 apart, Fig. 5B, direction D, in order to open up the slot S and form a wedge-shaped space W. When the spreading arms 52a,b have been brought to the predetermined clearance and have been locked in this state, and it has been established that the wedge-shaped space now made in the bone does in fact meet the requirements, placement of the implant 1 can proceed.

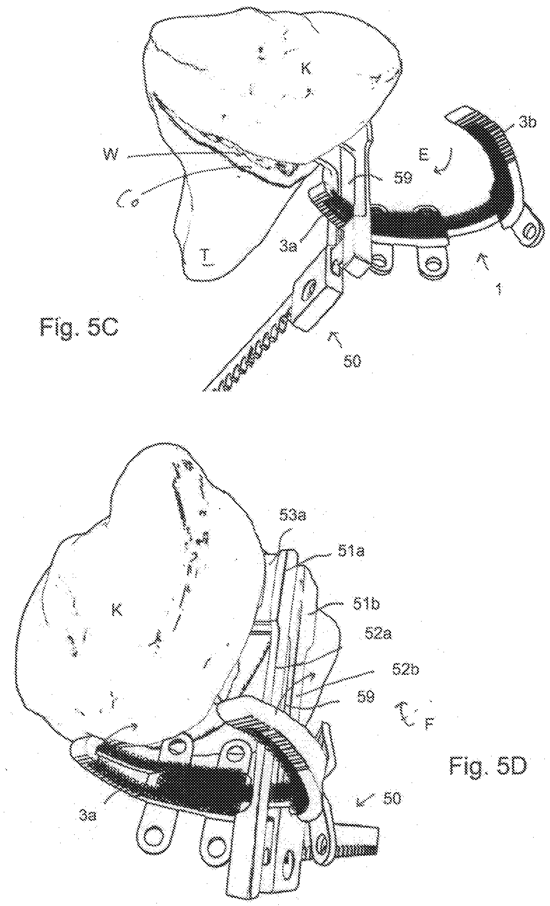

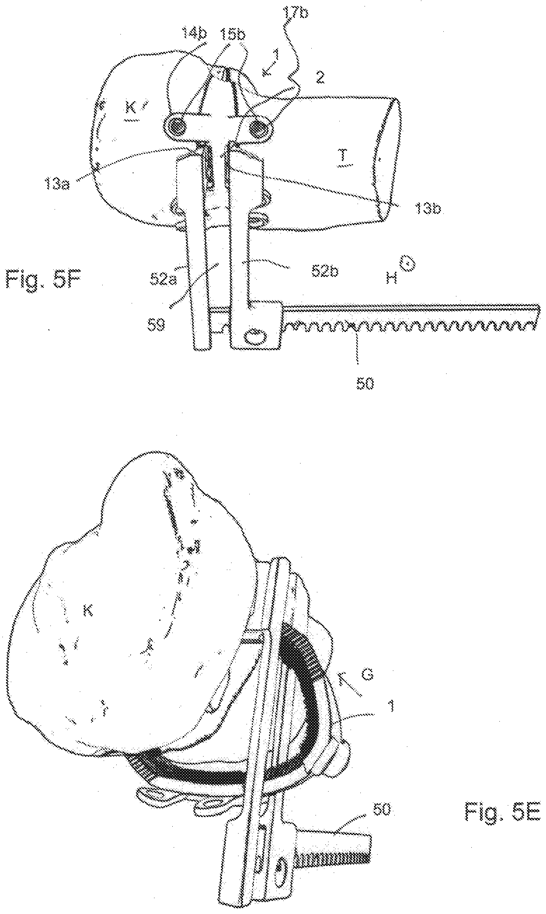

For this, the implant 1 is rotated (direction E, Fig. 5C) with arm 3a in the gap 59 that has been formed between the spreading arms 52a,b. When the state in Fig. 5D is reached and the middle portion 2 is located in the gap 59, the implant can be tilted toward the bone, direction F, Fig. 5D. The diagonal of the cross section of the middle portion 2 is smaller than the height of the gap 59. Then the implant 1 can be pushed in direction G, Fig. 5 E and rotated between the spreading arms 52a,b. The arm ends 9a,b enter the wedge-shaped space W, followed by the rest of the arms 3a,b. A hammering tool is optionally used.

The asymmetric teeth 10a,b contribute to keeping the implant in place. Even after the operation they may help to prevent pushing out of the implant as a result of axial loading in the leg. The lips 14a,b,c come up against the external surface of the bone, Fig. 5F. The implant is fastened to the bone with bone screws 17 through holes 15a,b,c. The precise positioning of these holes, determined by the made-to-measure drill guide 20, ensures correct placement of the implant. Then the bone spreader 50 can be removed, by pulling it back, direction H, Fig. 5F. The recesses 13a,b facilitate passage of the spreading jaws 53a,b.

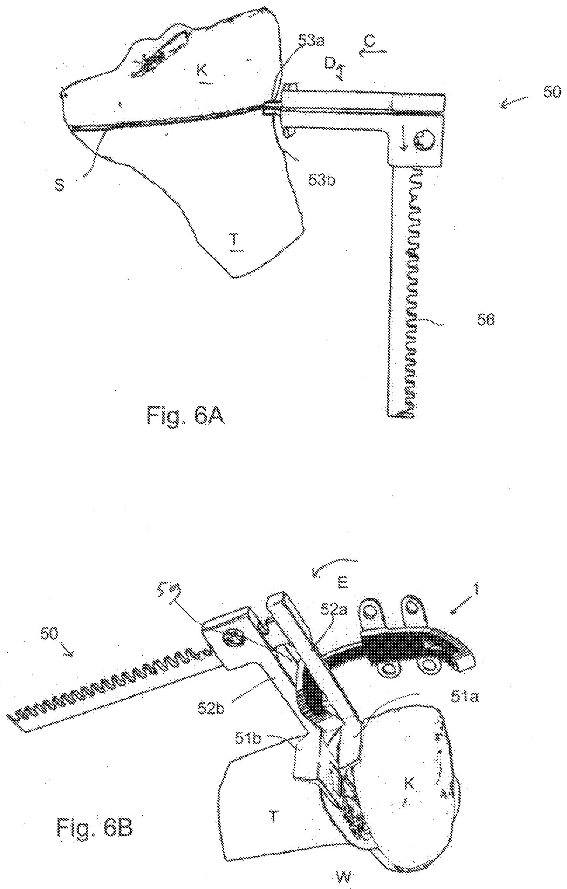

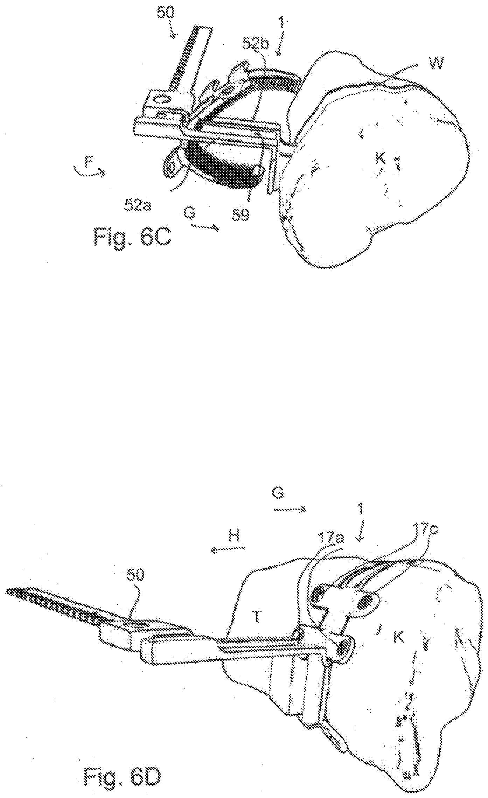

Figs. 6A-D show a comparable process with application of the bone spreader from Fig. 3C.

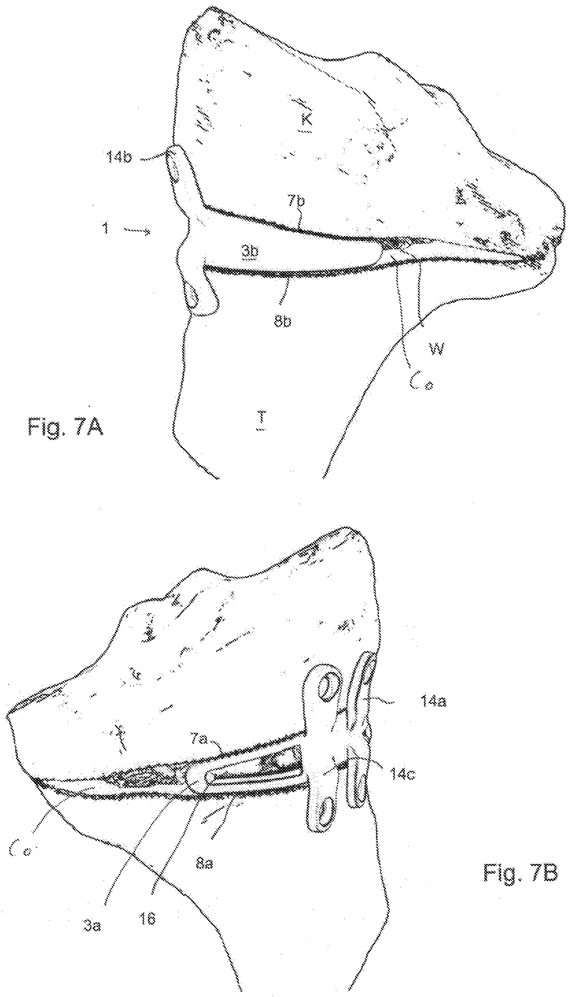

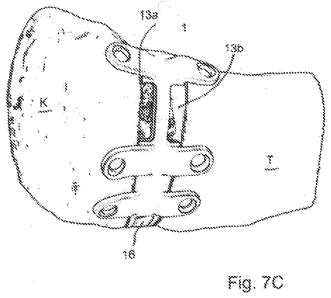

The result is illustrated in Figs. 7A-C. Via the recesses 13a,b and via the passage 16, a bone growth promoting agent can be introduced, such as minerals, growth factors, or stem cells. The porous layer 11 is in abutment with the cortex in the cut surfaces, so that bone ingrowth into this layer can take place.

Mainly the cortical part of the bone is supported, wherein the central medullar portion remains free. In this way - in the case of the knee joint - later insertion of a knee prosthesis, with extensions to the medullar canal, is not hampered.

The invention thus provides an implant that allows bone grafts or bone substitutes to be applied both before and after application of the implant.

The invention provides an implant that allows distraction of the bone segments with a segment retractor and insertion of the implant between the jaws of the retractor.

The invention provides an implant that takes the vertical loading directly without being dependent on the strength of osteosynthesis screws.

The implant will remain at a safe distance from the cortex that has not been sawn through, and the marrow cavity remains free for a future total knee prosthesis.

The invention(s) is/are not limited in any way to the embodiments shown and described in the drawings and description. The above description is employed to illustrate the action of preferred embodiments of the invention, and not to limit the scope of the invention.

## Claims

1. An implant (1) for placement in a wedge-shaped space in a bone obtained by osteotomy, wherein the implant is made from one whole and comprises a middle portion (2) and two arms (3a, 3b) connected thereto, extending from opposite sides of the middle portion, wherein the middle portion and the two arms define a U shape, wherein the arms each have an upper edge (7a, 7b) and a lower edge (8a, 8b) for engaging with the cut surfaces of the bone that define the wedge-shaped space, and that converge in a direction away from the middle portion, **characterized in that** the middle portion has an upper edge (12a) that is located lower than the upper edge of the adjacent portions of the arms and has a lower edge (12b) that is located higher than the lower edge of the adjacent portions of the arms for forming a recess (13a) above the middle portion and a recess (13b) below the middle portion.

2. The implant as claimed in claim 1, wherein the distance from the upper edge of the middle portion to the upper edge of the adjacent portions of the arms and the distance from the lower edge of the middle portion to the lower edge of the adjacent portions of the arms are approximately equal.

3. The implant as claimed in claim 1 or 2, wherein the middle portion and the adjacent portions of the arms define an H shape, wherein the middle portion forms the bridge of the H.

4. The implant as claimed in claim 1, 2 or 3, wherein the arms in the portions adjacent to the middle portion are provided with pairs of fastening lips (14a, 14b) formed as one whole therewith, extending transversely from the arms, respectively upwards and downwards, which are provided with holes (15a, 15b) for fasteners for securing the implant on the cortex of the bone.

5. The implant as claimed in claim 4, wherein at least one of the arms is provided with a second pair of fastening lips (14c) that is located closer to the end of the respective arm.

6. The implant as claimed in claim 4 or 5, wherein the fastening lips are displaced relative to the respective arm, in a direction turned away from the space (4) enclosed by the arms.

7. The implant as claimed in one of the preceding claims, wherein the arms have an internal surface (6) and an external surface (5), wherein the external surface of the arms is convex, considered in the direction of extension of the arms.

8. The implant as claimed in claim 7, wherein the internal surface of the arms is concave, considered in the direction of extension of the arms.

9. The implant as claimed in one of the preceding claims, wherein at least one arm, preferably each arm, on the internal surface facing the other arm is provided with a porous structure with interconnecting pores, allowing ingrowth of bone tissue.

10. The implant as claimed in claim 9, wherein the porous structure extends to at least one of the upper edge and lower edge of the respective arm.

11. The implant as claimed in one of the preceding claims, wherein the middle portion, on the internal surface facing the space delimited by the arms, is provided with a porous structure that allows ingrowth of bone tissue.

12. The implant as claimed in one of the preceding claims, wherein at least one of the arms is provided with a passage (16) to the space delimited by the arms, located between the upper edge passing through there and the lower edge passing through there.

13. An assembly of an implant as claimed in one of the preceding claims and a bone spreader (50), wherein the bone spreader has two spreading arms (51a, 51b) and spreading jaws (53a, 53b) located on the end thereof for forcing the cut surfaces of the bone apart, wherein the recesses above and below the middle portion are dimensioned to provide space for the respective spreading arms.

14. The assembly as claimed in claim 13, wherein the recesses above and below the middle portion are dimensioned for the spreading jaws to pass through them.

15. The assembly as claimed in claim 14, wherein the width of the recesses is greater than the width of the spreading arms and the width of the spreading jaws.

16. The assembly as claimed in claim 13, 14 or 15, wherein the spreading arms are joined together by a hinge (54), wherein the implant in at least one of the arms has a largest height (in particular the distance between the ends of two lips located opposite each other) that is less than the distance between the hinge and the spreading jaws.

## Patentansprüche

1. Implantat (1) zur Platzierung in einem durch Osteotomie erhaltenen keilförmigen Raum in einem Knochen, wobei das Implantat aus einem Ganzen besteht und einen Mittelabschnitt (2) und zwei damit verbundene Arme (3a, 3b), die sich von gegenüberliegenden Seiten des Mittelabschnitts erstrecken, umfasst,
wobei der Mittelabschnitt und die beiden Arme eine U-Form definieren,
wobei die Arme jeweils eine Oberkante (7a, 7b) und eine Unterkante (8a, 8b) zum Eingriff mit den Schnittflächen des Knochens aufweisen, die den keilförmigen Raum definieren und die in einer Richtung weg von dem Mittelabschnitt konvergieren,
**dadurch gekennzeichnet, dass** der Mittelabschnitt eine Oberkante (12a) aufweist, die tiefer als die Oberkante der benachbarten Abschnitte der Arme angeordnet ist, und eine Unterkante (12b) aufweist, die höher als die Unterkante der benachbarten Abschnitte der Arme angeordnet ist, um eine Vertiefung (13a) über dem Mittelabschnitt und eine Vertiefung (13b) unter dem Mittelabschnitt zu bilden.

2. Implantat nach Anspruch 1, wobei der Abstand von der Oberkante des Mittelabschnitts zu der Oberkante der benachbarten Abschnitte der Arme und der Abstand von der Unterkante des Mittelabschnitts zu der Unterkante der benachbarten Abschnitte der Arme etwa gleich sind.

3. Implantat nach Anspruch 1 oder 2, wobei der Mittelabschnitt und die angrenzenden Abschnitte der Arme eine H-Form definieren, wobei der Mittelabschnitt die Brücke des H bildet

4. Implantat nach Anspruch 1, 2 oder 3, wobei die Arme in den dem Mittelabschnitt benachbarten Bereichen mit Paaren von als Ganzes damit gebildeten Befestigungslippen (14a, 14b) versehen sind, die quer von den Armen nach oben bzw. unten verlaufen und mit Löchern (15a, 15b) für Befestigungselemente zur Befestigung des Implantats an der Rinde des Knochens versehen sind.

5. Implantat nach Anspruch 4, wobei wenigstens einer der Arme mit einem zweiten Paar von Befestigungslippen (14c) versehen ist, das näher an dem Ende des entsprechenden Arms angeordnet ist.

6. Implantat nach Anspruch 4 oder 5, wobei die Befestigungslippen relativ zu dem entsprechenden Arm in einer Richtung versetzt sind, die von dem Raum (4), der von den Armen umschlossenen wird, abgewandt ist.

7. Implantat nach einem der vorstehenden Ansprüche, wobei die Arme eine Innenfläche (6) und eine Außenfläche (5) aufweisen, wobei die Außenfläche der Arme in der Erstreckungsrichtung der Arme betrachtet konvex ist.

8. Implantat nach Anspruch 7, wobei die Innenfläche der Arme in der Erstreckungsrichtung der Arme betrachtet konkav ist.

9. Implantat nach einem der vorstehenden Ansprüche, wobei wenigstens ein Arm, vorzugsweise jeder Arm, auf der dem anderen Arm zugewandten Innenfläche mit einer porösen Struktur mit miteinander verbundenen Poren versehen ist, die Einwachsen von Knochengewebe erlauben.

10. Implantat nach Anspruch 9, wobei sich die poröse Struktur bis zu wenigstens einer von der Oberkante und der Unterkante des entsprechenden Arms erstreckt.

11. Implantat nach einem der vorstehenden Ansprüche, wobei der Mittelabschnitt auf der Innenfläche, die dem durch die Arme begrenzten Raum zugewandt ist, mit einer porösen Struktur versehen ist, die Einwachsen von Knochengewebe ermöglicht.

12. Implantat nach einem der vorstehenden Ansprüche, wobei wenigstens einer der Arme mit einem Durchgang (16) zu dem von den Armen begrenzten Raum versehen ist, der zwischen der dort hindurchgehenden Oberkante und der dort hindurchgehenden Unterkante angeordnet ist.

13. Anordnung eines Implantats nach einem der vorstehenden Ansprüche und eines Knochenspreizers (50), wobei der Knochenspreizer zwei Spreizarme (51a, 51b) und Spreizbacken (53a, 53b) aufweist, die an seinem Ende angeordnet sind, um die Schnittflächen des Knochens auseinanderzudrücken, wobei die Vertiefungen oberhalb und unterhalb des Mittelabschnitts bemessen sind, Platz für die entsprechenden Spreizarme bereitzustellen.

14. Anordnung nach Anspruch 13, wobei die Vertiefungen oberhalb und unterhalb des Mittelabschnitts zum Hindurchgehen der Spreizbacken bemessen sind.

15. Anordnung nach Anspruch 14, wobei die Breite der Vertiefungen größer als die Breite der Spreizarme und die Breite der Spreizbacken ist.

16. Anordnung nach Anspruch 13, 14 oder 15, wobei die Spreizarme durch ein Gelenk (54) miteinander verbunden sind,
wobei das Implantat an wenigstens einem der Arme eine größte Höhe (insbesondere den Abstand zwischen den Enden von zwei einander gegenüberliegender Lippen) aufweist, die kleiner als der Abstand zwischen dem Gelenk und den Spreizbacken ist.

## Revendications

1. Implant (1) destiné à être placé dans un espace en forme de coin d'un os obtenu par ostéotomie, l'implant étant constitué d'un seul tenant et comprenant une partie médiane (2) et deux bras (3a, 3b) reliés à celle-ci, s'étendant à partir de côtés opposés de la partie médiane,
la partie médiane et les deux bras définissant une forme en U,
les bras présentant chacun un bord supérieur (7a, 7b) et un bord inférieur (8a, 8b) pour coopérer avec les surfaces coupées de l'os qui définissent l'espace en forme de coin, et qui convergent dans une direction opposée à la partie médiane,
**caractérisé en ce que** la partie médiane présente un bord supérieur (12a) situé plus bas que le bord supérieur des parties adjacentes des bras et un bord inférieur (12b) situé plus haut que le bord inférieur des parties adjacentes des bras pour former un évidement (13a) au-dessus de la partie médiane et un évidement (13b) en dessous de la partie médiane.

2. Implant selon la revendication 1, la distance du bord supérieur de la partie médiane au bord supérieur des parties adjacentes des bras et la distance du bord inférieur de la partie médiane au bord inférieur des parties adjacentes des bras étant approximativement égales.

3. Implant selon la revendication 1 ou 2, la partie médiane et les parties adjacentes des bras définissant une forme en H, la partie médiane formant le pont du H.

4. Implant selon la revendication 1, 2 ou 3, les bras dans les parties adjacentes à la partie médiane étant pourvus de paires de lèvres de fixation (14a, 14b) formées d'un seul tenant avec ceux-ci, s'étendant transversalement à partir des bras, respectivement vers le haut et vers le bas, et pourvues de trous (15a, 15b) pour des attaches destinées à fixer l'implant sur le cortex de l'os.

5. Implant selon la revendication 4, au moins l'un des bras étant muni d'une seconde paire de lèvres de fixation (14c) située plus près de l'extrémité du bras respectif.

6. Implant selon la revendication 4 ou 5, les lèvres de fixation étant déplacées par rapport au bras respectif, dans une direction opposée à l'espace (4) entouré par les bras.

7. Implant selon l'une des revendications précédentes, les bras présentant une surface interne (6) et une surface externe (5), la surface externe des bras étant convexe, considérée dans la direction d'extension des bras.

8. Implant selon la revendication 7, la surface interne des bras étant concave, considérée dans la direction d'extension des bras.

9. Implant selon l'une des revendications précédentes, au moins un bras, de préférence chaque bras, sur la surface interne tournée vers l'autre bras, étant muni d'une structure poreuse avec des pores d'interconnexion permettant l'intégration du tissu osseux.

10. Implant selon la revendication 9, la structure poreuse s'étendant jusqu'à au moins l'un du bord supérieur et du bord inférieur du bras respectif.

11. Implant selon l'une des revendications précédentes, la partie médiane, sur la surface interne en regard de l'espace délimité par les bras, étant pourvue d'une structure poreuse permettant l'intégration du tissu osseux.

12. Implant selon l'une des revendications précédentes, au moins l'un des bras étant pourvu d'un passage (16) vers l'espace délimité par les bras, situé entre le bord supérieur le traversant et le bord inférieur le traversant.

13. Ensemble d'un implant selon l'une des revendications précédentes et d'un écarteur osseux (50), l'écarteur osseux comprenant deux bras d'écartement (51a, 51b) et des mâchoires d'écartement (53a, 53b) situées à leur extrémité pour forcer les surfaces coupées de l'os à s'écarter, les évidements au-dessus et au-dessous de la partie médiane étant dimensionnés pour fournir un espace pour les bras d'écartement respectifs.

14. Ensemble selon la revendication 13, les évidements au-dessus et au-dessous de la partie médiane étant dimensionnés pour que les mâchoires d'écartement les traversent.

15. Ensemble selon la revendication 14, la largeur des évidements étant supérieure à la largeur des bras d'écartement et à la largeur des mâchoires d'écartement.

16. Ensemble selon la revendication 13, 14 ou 15, les bras d'écartement étant reliés entre eux par une charnière (54),
l'implant dans au moins un des bras présentant une plus grande hauteur, notamment la distance entre les extrémités de deux lèvres situées en regard l'une de l'autre, qui est inférieure à la distance entre la charnière et les mâchoires d'écartement.
